Europäisches Patentamt

European Patent Office

Office européen des brevets

(19)

(11) Publication number: **0 320 103**
**A2**

# EUROPEAN PATENT APPLICATION

(21) Application number: 88310059.6

(22) Date of filing: 26.10.88

(51) Int. Cl.⁴: **C12N 15/00 , C07H 21/00**

(30) Priority: 29.10.87 JP 274388/87

(43) Date of publication of application:
**14.06.89 Bulletin 89/24**

(84) Designated Contracting States:
**DE FR GB**

(71) Applicant: **AJINOMOTO CO., INC.**
**5-8, Kyobashi 1-chome, Chuo-ku**
**Tokyo 104(JP)**

(72) Inventor: **Shibahara, Susumu Central**
**Research Laboratories**
**Ajinomoto Co., Inc. No. 1-1, Suzuki-cho**
**Kawasaki-ku Kawasaki-shi**
**Kanagawa-ken(JP)**
Inventor: **Morisawa, Hirokazu Central**
**Research Laboratories**
**Ajinomoto Co., Inc. No. 1-1, Suzuki-cho**
**Kawasaki-ku Kawasaki-shi**
**Kanagawa-ken(JP)**
Inventor: **Mukai, Sachiko c/o Central**
**Research Laboratories**
**Ajinomoto Co., Inc. No. 1-1, Suzuki-cho**
**Kawasaki-ku Kawasaki-shi**
**Kanagawa-ken(JP)**

(74) Representative: **Bond, Bentley George et al**
**HASELTINE LAKE & CO. Hazlitt House 28**
**Southampton Buildings Chancery Lane**
**London, WC2A 1AT(GB)**

(54) **Compounds for DNA deletion and oligomers used therefor as well as a method for use thereof.**

(57) There is provided a single-stranded linear polynucleotide for use in the deletion of DNA from a gene, having the general formula I or I′:

$$5'\text{-A-}(B)_m\text{-X-}(C)_n\text{-D-}3' \quad (I)$$

or

$$5'\text{-A-}(B)_m\text{-X-}(C)_n\text{-}3' \quad (I')$$

wherein A and D are each, independently, (i) a terminal DNA sequence as formed in one of the single strands of a double-stranded sequence of base-pairs at the cleavage site of a restriction enzyme recognition sequence or (ii) a DNA linker sequence from which a terminal DNA sequence as described in (i) above may be generated; wherein B and C are each, independently, chosen from (a) one of the single-stranded parts of a double-stranded sequence of deoxyribonucleotide base pairs which constitutes a restriction enzyme recognition sequence, optionally coupled at either end with an integral number of additional deoxyribonucleotides chosen from A, C, G and T, and (b) any sequence of deoxyribonucleotides, and m and n are each, independently, zero or an integer of from 1 to 5; with the proviso that, in the polynucleotide of general formula I or I′ there is present at least one sequence representing B or C which is in accordance with the definition given in (a) above; and wherein X includes a sequence of nucleotides which, in double-stranded form, is resistant to digestion by a

DNA nuclease.

Also provided is a double-stranded oligomer for use in DNA deletion, a method for producing a compound for DNA deletion and a method of DNA deletion using a DNA nuclease.

# COMPOUNDS FOR DNA DELETION AND OLIGOMERS USED THEREFOR AS WELL AS A METHOD FOR USE THEREOF REFERENCE TO RELATED APPLICATION

The present invention relates to novel compounds which are useful for the mass production of useful proteins, for improving the function of proteins and for clarifying the relationship between structure and function of useful proteins. The components are useful for rendering virulent RNA virus non-harmful, for the utilization of attenuated virus, for the establishment of effective therapeutic treatments. The present invention can further be used as a means for producing, for example, deletion mutants for the manipulation of DNA or as a means effective for sequencing. The invention also relates to the oligomers used therefor as well as to methods for use thereof.

In the field of genetic engineering, the deletion of a gene at a specific site thereof is an effective and widely used method. The method can be applied when, for example, the sequencing of a cloned gene is desired or when deletion mutants of a gene encoding an enzyme are to be produced and their activity is to be investigated by comparison with that of wild types or when the function of a genetic region that controls gene expression is to be investigated.

At present, two representative methods are known, namely a method using Bal 31 nuclease (see, for example, Proc. Natl. Acad. Sci., 79, 4298 (1982)), and a method which comprises treating with exonuclease III, then treating a single strand part with SI nuclease or Mung Bean nuclease and then subjecting to a Klenow fragment treatment (see, for example, Methods in Enzymolgy 100, 60 (1983)). In the former method, however, the deletion proceeds in both directions so that the method is not suitable for the production of deletion mutants in one direction only. Therefore to obtain the deletion mutants in one direction only, it is necessary to subject the shortened DNA fragments, obtained from a deletion process that proceeds in both directions, to subcloning.

In the latter case, it is necessary that two sites for restriction enzymes that generate the $5'$-protruding end and the $3'$-protruding end, respectively, exist at the sites to be deleted. In addition, deletion mutants from the $5'$-protruding end only can be obtained but those from the other end cannot be obtained. Furthermore, even if adaptor DNA fragments having desired two sites are artificially produced, it is necessary that the two new restriction sites be unique and suitable for the conditions described above. Accordingly, the restriction enzymes that can be used are restricted. Furthermore, as has been described above, the known method has the disadvantage that complicated treatments in combination with enzyme reactions are required.

## SUMMARY OF THE INVENTION

It is desired to develop a simple method for producing deletion mutants e.g. deletion mutants of a gene encoding functional protein, for the clarification of the mechanism of gene expression and for the sequencing of cloned genes.

According to the present invention, the deletion of a DNA fragment in only one direction is carried out using a chimeric oligomer. The present invention can be applied to produce independently deletion mutants in both directions from, for example, a cloned gene, depending upon necessity. The present invention can also be used for the purpose of simply deleting a certain DNA fragment from the end thereof.

The chimeric oligomer used herein can be prepared in a good yield in accordance with the known phosphoramdite method.

According to one aspect of the present invention, there is provided a single-stranded linear polynucleotide for use in the deletion of DNA from a gene, having the general formula I or I':

$$5'\text{-A-(B)}_m\text{-X-(C)}_n\text{-D-3}' \qquad (I)$$

or

$$5'\text{-A-(B)}_m\text{-X-(C)}_n\text{-3}' \qquad (I')$$

wherein A and D are each, independently, (i) a terminal DNA sequence as formed in one of the single strands of a double-stranded sequence of base-pairs at the cleavage site of a restriction enzyme recognition sequence or (ii) a DNA linker sequence from which a terminal DNA sequence as described in (i) above may be generated;

wherein B and C are each, independently, chosen from (a) one of the single-stranded parts of a double-stranded sequence of deoxyribonucleotide base pairs which constitutes a restriction enzyme recognition sequence, optionally coupled at either end with an integral number of additional deoxyribonucleotides chosen from A, C, G and T, and (b) any sequence of deoxyribonucleotides; with the proviso that, in the polynucleotide of general formula I or I′ there is present at least one sequence representing B or C which is in accordance with the definition given in (a) above;

wherein X includes a sequence of nucleotides which, in double-stranded form, is resistant to digestion by a DNA nuclease;

and wherein m and n are each, independently, zero or an integer of from 1 to 5.

According to a second aspect of the invention, there is provided a single-stranded linear polynucleotide for use in the deletion of DNA from a gene, having the general formula I or I′:

$$5′\text{-A-(B)}_m\text{-X-(C)}_n\text{-D-3}′ \quad \text{(I)}$$

or

$$5′\text{-A-(B)}_m\text{-X-(C)}_n\text{-3}′ \quad \text{(I′)}$$

wherein A and D are independently chosen from

(i) a single-stranded linker of deoxyribonucleotides having the general formula II or II′

$$R_5\text{-W-}R_6 \quad \text{(II) or W} \quad \text{(II′)}$$

in which each of $R_5$ and $R_6$ represents a sequence of the same length comprising 1, 2 or 3 nucleotides chosen from G (guanine) and C (cytosine) and W is a sequence comprising from 4 to 6 nucleotides chosen from G, C, A (adenine) and T (thymidine) or W is a sequence comprising 7 nucleotides chosen from G, C, A and T, the terminal nucleotides of which sequence are chosen from G and C; with the proviso that the sequence of nucleotides in the linker of general formula II or II′ is chosen such that, when it is base-paired with its complementary DNA sequence, there is formed a palindromic sequence of base pairs; and

(ii) a terminal DNA sequence of from one to six nucleotides formed by cleavage between two adjacent nucleotides of sequence W as defined above;

wherein B and C are independently chosen from

(a) a single-stranded length of deoxyribonucleotides of sequence W as defined in relation to general formula II above, optionally coupled at either end with an integral number of additional deoxyribonucleotides chosen from A, C, G and T; and

(b) any sequence of deoxyribonucleotides; with the proviso that in the polynucleotide of general formula I or I′ there is present at least one sequence representing B or C which is in accordance with the definition given in (a) above;

wherein X represents a sequence of nucleotides which, in double-stranded form, is resistant to digestion by a DNA nuclease;

and m and n are each, independently, zero or an integer of from 1 to 5.

According to a third aspect of the present invention there is provided a single-stranded polynucleotide for use in the deletion of DNA from a gene, having the general formula I or I′:

$$5′\text{-A-(B)}_m\text{-X-(C)}_n\text{-D-3}′ \quad \text{(I)}$$

or

$$5′\text{-A-(B)}_m\text{-X-(C)}_n\text{-3}′ \quad \text{(I′)}$$

wherein A and D are each, independently, represented by a linker sequence (of general formula II or II′ below) or a terminal DNA sequence (of general formula III below) as formed in one of the single strands of a double-stranded sequence of base pairs at the cleavage site of a restriction enzyme recognition sequence; B and C are each, independently, represented by (a) a single-stranded part (of general formula II below) of a double-stranded sequence of deoxyribonucleotide base pairs which constitutes a restriction enzyme recognition sequence, optionally coupled at either end with an integral number of additional deoxyribonucleotides chosen from A, C, G and T or (b) any sequence of deoxyribonucleotides; with the proviso that, in the compound of formula I or I′ there is present at least one sequence representing B or C which is in accordance with the definition given in (a) above; wherein X represents a sequence of nucleotides which, in double-stranded form, is resistant to digestion by a DNA nuclease; and m and n are each, independently, zero, or an integer of from 1 to 5;

said linker sequence in general formula I being the same as the single-stranded part of a restriction enzyme recognition sequence (general formula II) or represented by general formula II′:

$$W \quad \text{(II)}$$

$$R_5\text{-W-}R_6 \quad \text{(II′)}$$

wherein $R_5$ and $R_6$ each represents a sequence of the same length comprising 1, 2 or 3 deoxyribonucleotides composed of G (guanine) or C (cytosine); and $R_5$ and $R_6$ are chosen to be complementary when the sequence represented by general formula II′ forms double strands;

said single-stranded part of a restriction enzyme recognition sequence of general formulae II being selected from the following 8 sequence groups:

$$R_1R_2R_3R_4, \qquad R_1R_2NR_3R_4,$$
$$AR_1R_1R_3R_4T, \qquad CR_1R_2R_3R_4G,$$
$$GR_1R_2R_3R_4C, \qquad TR_1R_2R_3R_4A,$$
$$CR_1R_2NR_3R_4G \text{ and } GR_1R_2NR_3R_4C$$

wherein the sequential order of $R_1$, $R_2$, $R_3$ and $R_4$ comprises, irrespective of the presence or absence of N, the following 16 sequence groups, reading from the left:

AATT, ACGT, AGCT, ATAT,
CATG, CCGG, CGCG, CTAG,
GATC, GCGC, GGCC, GTAC,
TATA, TCGA, TGCA, TTAA,

and N represents a deoxynucleotide unit of G, C, A (adenine) and T (thymidine),

said single-stranded part of the terminal DNA sequence generated at the cleavage site of a restriction enzyme recognition site being composed of a sequence group represented by general formula III:

$R_1R_2R_3R_4$,
$R_2R_3R_4$, $R_3R_4$, $R_4$,
$R_2NR_3R_4$, $NR_3R_4$, $R_3R_4$, $R_4$
$R_1R_2R_3R_4Y$, $R_2R_3R_4Y$, $R_3R_4Y$, $R_4Y$, Y,
$R_1R_2NR_3R_4Z$, $R_2NR_3R_4Z$, $NR_3R_4Z$, $R_3R_4Z$, $R_4Z$, Z,
$R_1R_2R_3$, $R_1R_2$, $R_1$,
$R_1R_2NR_3$, $R_1R_2N$, $R_1R_2$, $R_1$,
$YR_1R_2R_3R_4$, $YR_1R_2R_3$, $YR_1R_2$, $YR_1$, Y
$ZR_1R_2NR_3R_4$, $ZR_1R_2NR_3$, $ZR_1R_2N$, $ZR_1R_2$, $ZR_1$, Z

in which Y represents a deoxyribonucleotide unit of G, C, A and T; Z represents a deoxyribonucleotide unit of G and C, and N has the same definition as described above.

It is to be appreciated that where m and/or n is greater than 1, the repeating B or C unit need not necessarily be the same. Thus, for example, a compound in accordance with the invention could have several different B and C regions.

The region X preferably includes a sequence which, in double-stranded form, is resistant to Bal 31 nuclease. For example, the region X may include one or more ribonucleotide or derivative thereof which is resistant to Bal 31 nuclease, such as a $2'$-O-methylribonucleotide.

According to a fourth aspect of the present invention, there is provided a compound for DNA deletion comprising two or more polynucleotides represented by he general formula I or I' above linked in tandem.

According to a fifth aspect of the invention, there is provided a double-stranded oligomer comprising a primary strand of general formula I or I' above base-paired with its complementary DNA strand in which the region of he complementary strand which is base-paired with the X-region of the primary strand optionally comprises a sequence which is resistant to digestion by a DNA nuclease;

wherein, if the sequence of nucleotides represented by A and D in the primary strand of formula I or I' is a terminal DNA sequence, the corresponding terminal sequence of the complementary strand of the oligomer is chosen such that the respective end of the oligomer corresponds to the end formed at the cleavage site of a restriction enzyme recognition sequence. One or more of such oligomer may be linked in tandem.

According to a sixth aspect, there is provided a method for producing a compound for DNA deletion comprising generating, from double stranded monomer in accordance with the fifth aspect of the present invention which has one or two terminal linker sequence, an oligomer according to the fifth aspect above in which the respective end or ends of the oligomer correspond, independently, to the end formed at the cleavage site of a restriction enzyme recognition sequence, and subsequently ligating one or both of the ends of the thus formed oligomer to the corresponding end or ends of a desired double-stranded DNA fragment.

According to a seventh aspect of the invention there is provided a method for producing a compound for DNA deletion comprising ligating, to the corresponding end or ends of a desired double-stranded DNA fragment, one or both of the ends of an oligomer in accordance with the fifth aspect of the invention in which one or both of the ends of the oligomer correspond, independently, to the end formed at the cleavage site of a restriction enzyme recognition sequence.

Finally, according to an eighth aspect of the invention there is provided a method of DNA deletion comprising subjecting to deletion by a DNA nuclease a compound as produced by the method of the sixth or seventh aspect of this invention in which one only of the ends of the oligomer is ligated with the corresponding end of the desired double-stranded DNA fragment; or, if both ends of the oligomer are ligated with the corresponding ends of the desired double-stranded DNA fragment, first cleaving the compound as produced by the method of the sixth or seventh aspect of this invention with a restriction enzyme at one of the regions A, B, C, or D, containing a restriction site, and second subjecting the resulting derivative to deletion by a DNA nuclease.

Reference will be made hereinbelow, by way of example, to the drawings in which:

Figure 1 illustrates a reaction scheme described in detail hereinbelow.

Figure 2 illustrates a cloned DNA gene described in detail hereinbelow.

Figure 3 illustrates the plasmid pUC18.

Figure 4 illustrates a reaction scheme described in detail hereinbelow.

Figure 5 shows an autoradiograph obtained by 20% polyacrylamide gel electrophoresis of the ligation product described below, wherein lane 1 indicates the ligation product of double strand (1) and lane 2 indicates likewise that of double strand (8). A and B each represent a monomer.

Figure 6A and 6B show autoradiographs obtained by 20% polyacrylamide gel electrophoresis of a product treated with a restriction enzyme, as described below, wherein (1) to (8) indicate ligation products which are obtained by treating the respective dimers (including monomers) with BamH I, Sma I, Hinc II and Sac I and the respective lane numbers are as follows:

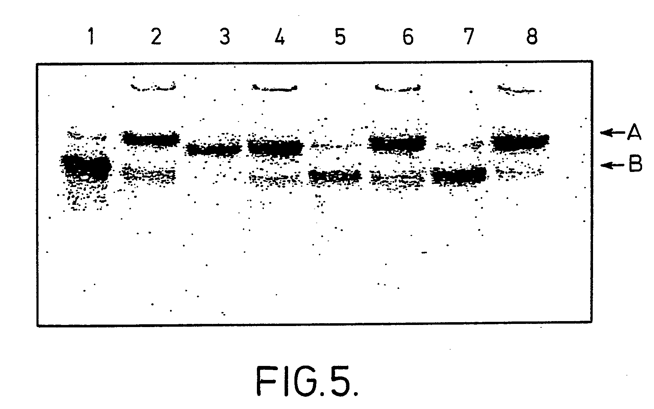

```
Same sample as in Figure 5 :   lanes 1, 3, 5 and 7
BamH I treated product      :   lanes 9, 13, 17, 21,
                                25, 27, 29, 31
Sma I           "           :  ·lanes 10, 14, 18,
                                22, 26, 28, 30, 32
Hinc II         "           :   lanes 11, 15, 19, 23
Sac I           "           :   lanes 12, 16, 20, 24
```

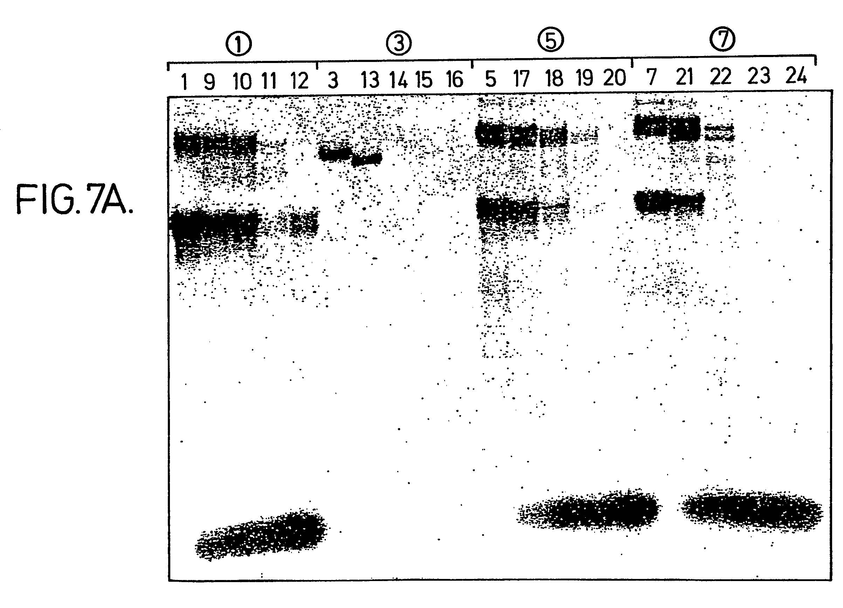

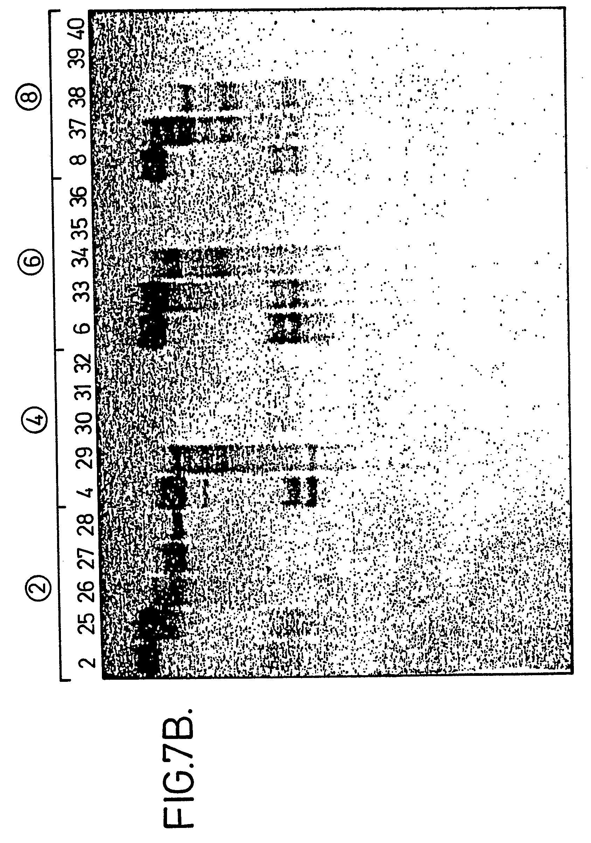

Figures 7A and 7B show autoradiographs obtained by 20 % polyacrylamide electrophoresis of a product, as described below, of mixed oligomers treated with Bal 31 nuclease with passage of time, wherein (1) to (8) indicate the ligation products shown in Figure 5. The lane numbers are as follows:

```
Same sample as in Figure 5 :  lanes 1, 2, 3, 4, 5,
                                6, 7, 8
       0 Minute            :   lanes 1, 3, 5, 7, 2,
                                4, 6, 8
       1 Minute            :   lanes 9, 13, 17, 21,
                                25, 29, 33, 37
       5 Minutes           :   lanes 10, 14, 18,
                                22, 26, 30, 34, 38
      10 Minutes           :   lanes 11, 15, 19,
                                23, 27, 31, 35, 39
      25 Minutes           :   lanes 12, 16, 20,
                                24, 28, 32, 36, 40
```

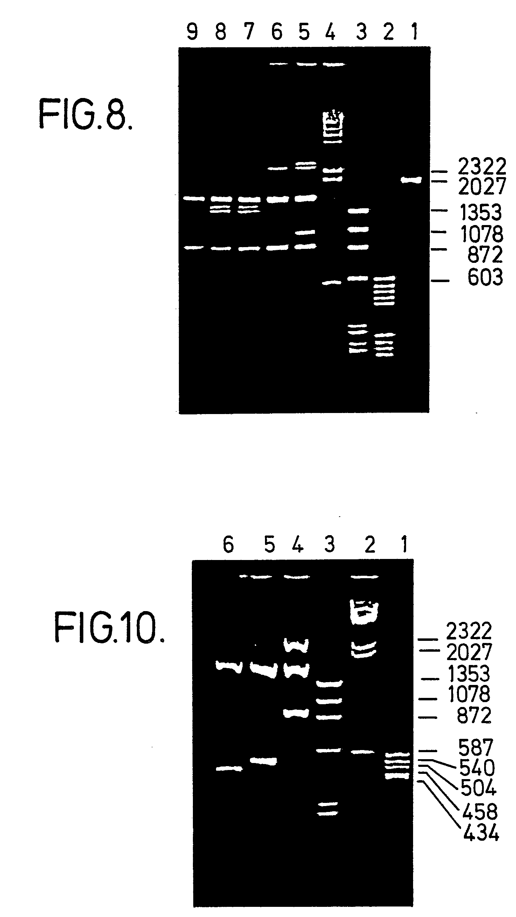

Figure 8 shows a gel stained with ethidium bromide after 1.5% agarose gel electrophoresis of digested fragments of the products of plasmid pUC18R treated Bal 31 nuclease with passage of time, as described below. Lane 1 indicates the pUC18 plasmid and lanes 2, 3 and 4 indicate pBR 322 DNA treated with Hae II, ø x 174 DNA treated with Hae III and lambda DNA treated with Hind III, respectively, and used as markers. Lanes 5, 6, 7, 8 and 9 indicate samples for 0 minute, 10 minutes, 30 minutes, 45 minutes and 90 minutes, respectively.

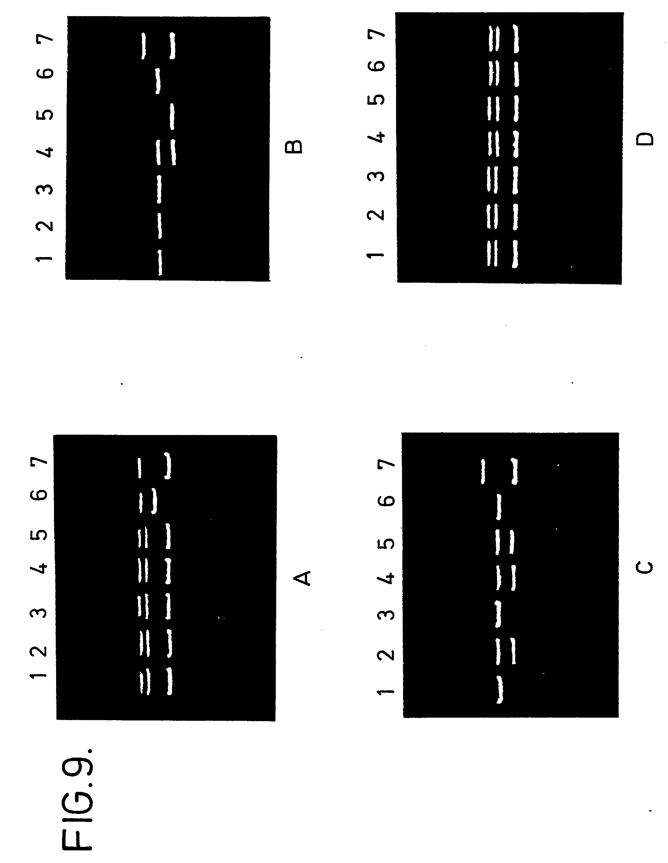

Figure 9 shows gels obtained by treating deletion clones obtained by treating plasmid pUC18R with Bal 31 nuclease with restriction enzyme, examining the presence or absence of its site by 1.2% agarose gel electrophoresis and ethidium bromide staining. Lane 1 relates to that treated with Hind III and lanes 2, 3, 4, 5 and 6 relate to those treated with Sph I, Pst I, Hinc II, Xba I and EcoR I, respectively, and lane 7 relates to the intact clone. Gels A, B, C and D indicate a pattern in which the EcoR I site alone remains, a pattern in which the EcoR I and Hind III to Hinc II sites remain, a pattern in which all of the sites remain and a pattern in which none of the sites remains, respectively.

Figure 10 shows a gel stained with ethidium bromide after 2% agarose gel electrophoresis of the fragments which were generated by EcoR I and Sca I-double digestions of 2 deletion clones that were obtained by Bal 31 treatment of pUC18R for 45 and 60 minutes, respectively. Lanes 1 to 3 indicate size markers, lane 4 indicates a Bal 31 untreated sample for 0 minute, lane 5 indicates the sample from the clone which was treated with Bal 31 for 45 minutes and lane 6 indicates the sample from the clone which was treated with Bal 31 for 60 minutes.

The present invention will now be described, by way of example, with reference to the drawings and Examples.

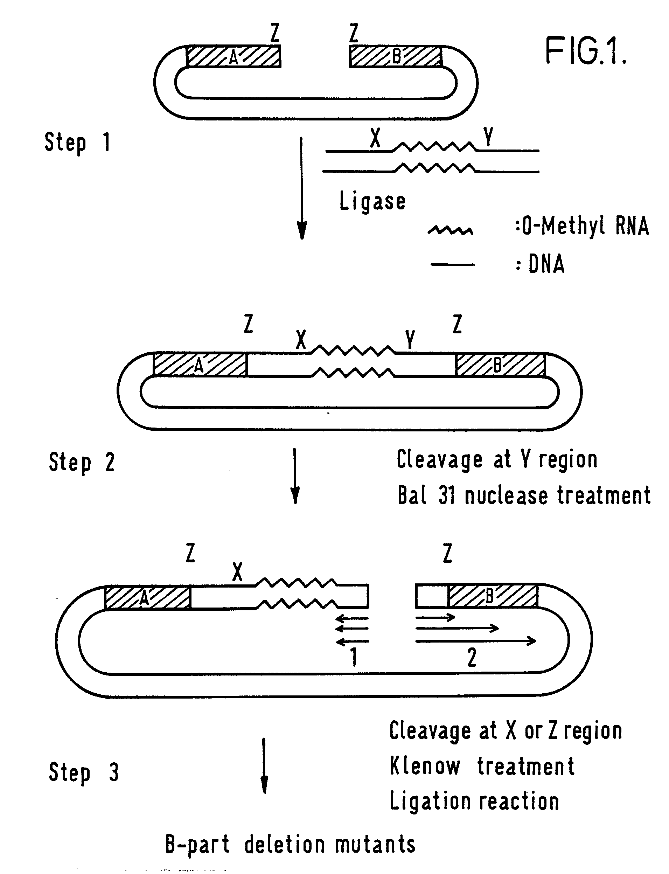

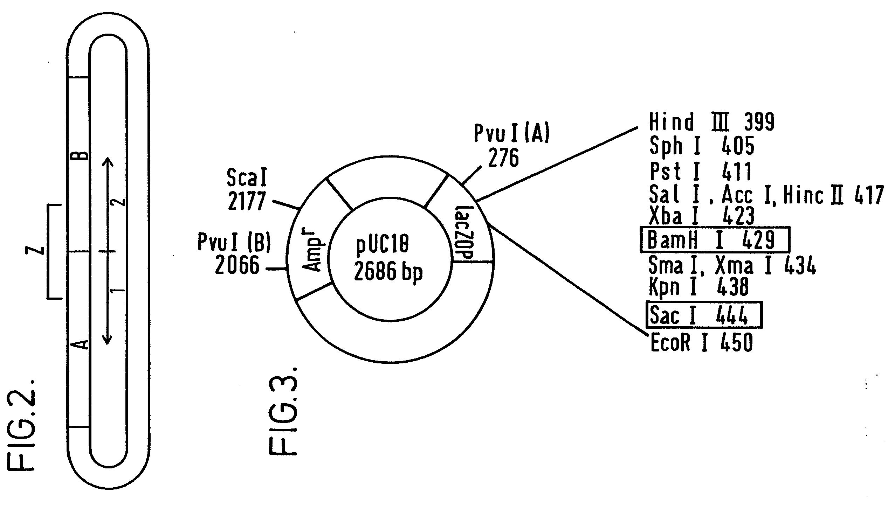

In the case of Fig. 1, it is assumed that at least one unique restriction site exists at the Z region in a cloned DNA gene consisting of both A and B portions (as is shown in Fig. 2).

According to the two known methods described above, deletion takes place in both directions as shown by arrows 1 and 2 in the case of the first method (Figure 2). In the case of the second method, the direction of arrow 1 or 2 is determined by two or more restriction sites.

According to the present invention, the following takes place: cleavage with the restriction enzyme takes place in the Z region; ligation of double stranded oligomer consisting of, for example, DNA-O-methyl RNA chimeric oligomer with the same sites as above restriction enzyme (Fig. 1, Step 1); cleavage with restriction enzyme in the Y region subsequent thereto (in which the subsequent cleavage with restriction enzyme can be omitted in the case of ligation at one end only of the chimeric oligomer); Bal 31 nuclease treatment, sampling with passage of time and pooling them result in the deletion in the B region of the gene. With respect to the direction of arrow 1, the Bal 31 exonuclease reaction is discontinued or its reaction rate becomes slow at the O-methyl RNA site. Therefore, no deletion occurs in the A region of the gene (Fig. 1, Step 2). Next, a group of deletion mutants in the B region of the gene are obtained by cleavage in the X region or Z region, and by Klenow treatment and ligation subsequent thereto.

In Fig. 1, when two different restriction enzymes are used for cleavage in the Z region, the chimeric oligomer with the corresponding sites can be employed, whereby the direction of the chimeric oligomer to be inserted can be chosen. Furthermore, by cleaving in either the Y or X region, it is possible to cause deletion separately in each direction. Furthermore, when using one restriction enzyme for cleavage in the Z region, the direction of the chimeric oligomer to be inserted cannot be chosen; however, by cleaving at either X or Y, deleted mutants towards only one direction each can be obtain simultaneously and separated by an appropriate screening method.

In order to make this new deletion method possible, the following should be confirmed by experiment;

Step 1 :     It should be confirmed that the ligation reaction can be effected in the DNA part of chimeric oligomer.

Steps 2 and 3 :     It should be confirmed that cleavage with the restriction enzyme can be effected in the DNA part of the chimeric oligomer. In the digestion reactions with Bal 31 nuclease, the reaction is discontinued at O-methyl RNA part or the reaction rate becomes markedly slow.

In order to carry out the foregoing experiments, chimeric oligomers V and VI were prepared:

5'-GATCCGmGmAmUmCmCmCmGGGGTCGACGAGCT-3'  (V)

3'-GCmCmUmAmGmGmGmCCCCAGCTGC-5'      (VI)

BamH I     BamH I

Sma I     Sal I     Sac I

Hinc II

For preparation of the chimeric oligomers, the phosphoramdite method using a beta-cyanoethyl group as a protecting group of the phosphite moiety was employed. Good results were obtained (see Example 1 below).

Thus (referring to Table 1 below) $2'$-O-methyl-N$^4$-benzoylcytidine (Compound I), $2'$-O-methyl-N$^6$-benzoyladenosine (Compound II), $2'$-O-methyl-N$^2$-isobutyrylguanosine (Compound III) and $2'$-0methyluridine (Compound IV) were prepared, according to Nucleic Acids Symposium Series, No. 16, 165, 1985. The $5'$-hydroxy group of each compound was further protected with a dimethoxytrityl group (DMTr-) or a monomethoxytrityl group (MMTr-) to prepare Compounds Ia, IIa, IIIa and IVa. The $3'$-hydroxy group in Compounds Ia, IIa, IIIa and IVa was reacted with 2-cyanoethyl N,N-diisopropylchlorophosphoramide in a manner similar to the method described in Nucleic Acids Res., 15, 4403 (1987) to give Compounds Ib, IIb, IIIb and IVb. With respect to preparation of DNA part, commercially available reagents Ic, IIc, IIIc, IVc, IId and IVd, ABI Co., Ltd. were used. Using these reagents, Compounds V and VI were prepared in accordance with the method of Table 3A (see Example 1).

Furthermore, at the same time, DNA oligomers VII and VIII having the same sequence as the corresponding Compounds V and VI respectively were prepared in accordance with Tetrahedron Lett., 22, 245 (1983).

These compounds are represented as follows:

Compound V     5'——⋀⋀⋀⋀⋀⋀——3'

Compound VI     3'——⋀⋀⋀⋀⋀⋀——5'

Compound VII     5'————————3'

Compound VIII     3'———————— 5'

Next, Compounds V to VIII were labeled with a phosphate group by T4-polynucleotide kinase using gamma-$^{32}$P-ATP.

Subsequently, eight double stranded monomers (1) to (8) were prepared by annealing as shown below. Each monomer was subjected to a ligation reaction by T4-DNA ligase to form a dimer. It was confirmed by 20% polyacrylamide electrophoresis that the dimers were almost quantitively formed from the double stranded monomers ((2), (3), (4), (6) and (8)).

Double strands (1)

" (2)

" (3)

" (4)

" (5)

" (6)

" (7)

" (8)

o : represents labeled phosphate group.

Furthermore, in the case the double stranded monomers (1), (5) and (7), 2-0-methyl RNA is present adjacent one base at the binding site of the ligation reaction so that the yield of dimer was somewhat low but dimers could be obtained (Example 3 and Figure 5). From this, the litigation reaction proceeded in the Z region at the step under the aforesaid conditions and the conditions were satisfied.

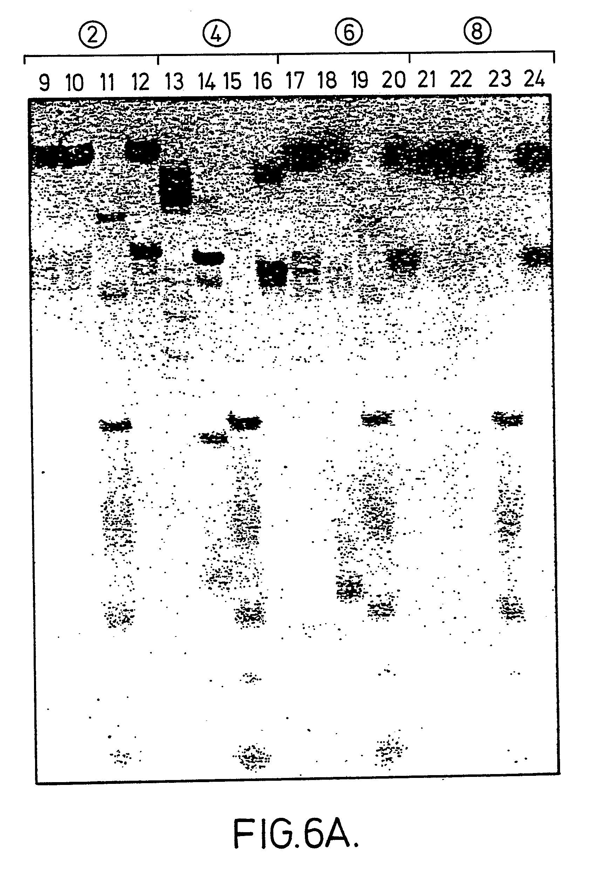

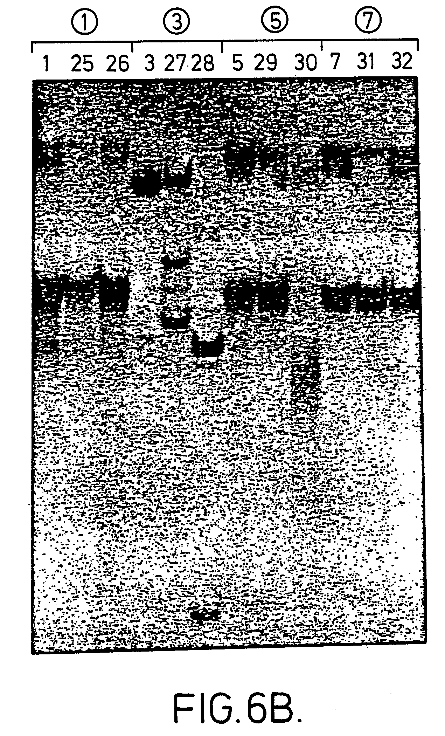

The resulting dimers (including monomers (1) to (8) and dimers thereof) were treated with BamH I, Sma I, Hinc II and Sac I, respectively. The cleavage reaction did not occur at the BamH I site in the $2'$-O-methyl RNA region in the case of the double strands composed of O-methyl RNA and its complementary O-methyl RNA or even though either strand is DNA, while it is unclear if the above result is affected by T being substituted with U or O-methyribose. However, with respect to the Sma I site, the cleavage occurred in both strands with regard to the combination in double strands (5) and (6), among six sequences in which three bases each of 2′-O-methyl RNA are contained in the recognition site. But double strands (1), (2), (7) and (8) were not cleaved, reflecting a slight change in the recognition site. With respect to BamH I, Hinc II and Sac I sites in the other DNA regions, cleavage occurred (see Example 5 and Figures 6A and 6B). From this, cleavage at the restriction enzyme site in the X or Y region of Steps 2 and 3 (Figure 1) proceeded and the conditions were satisfied.

Next, digestion with Bal 31 nuclease which is the most important principle of the present invention will be investigated using monomers (1) to (8) and dimers thereof.

The dimer (containing 20 to 30 pmoles converted to monomer) was reacted at 30°C for 0 to 25 minutes in 50 microlitres of a buffer for Bal 31 reaction consisting of Bal 31 (0.26 U) in 600 mM of NaCl. Each reaction product formed with the passage of time was analyzed on 20% polyacrylamide electrophoresis (Figures 7A and 7B).

The chimeric oligomers obtaining 2′-O-methyl RNA obviously resisted the digestion, as compared to the corresponding DNA dcuble strands (3) and (4);

notwithstanding the fact that the oligomer consisting of DNA strand alone completely disappeared for 1 to 5 minutes (cf. Figure 7, lanes 14 and 30), the oligomer containing 2′-O-methyl RNA remained even after 10 minutes and 15 minutes. In particular, in double strands (1) and (2) wherein double strands both consist of 2′-O-methyl RNA, a band of a size corresponding to the 2′-O-methyl RNA double strand part remained (cf. Figure 7, lanes 12 and 28).

These results show that the 2′-O-methyl RNA part, in double stranded forms, was resistant to Bal 31 nuclease digestion, indicating that the conditions necessary in step 2 of Figure 1 are suitable.

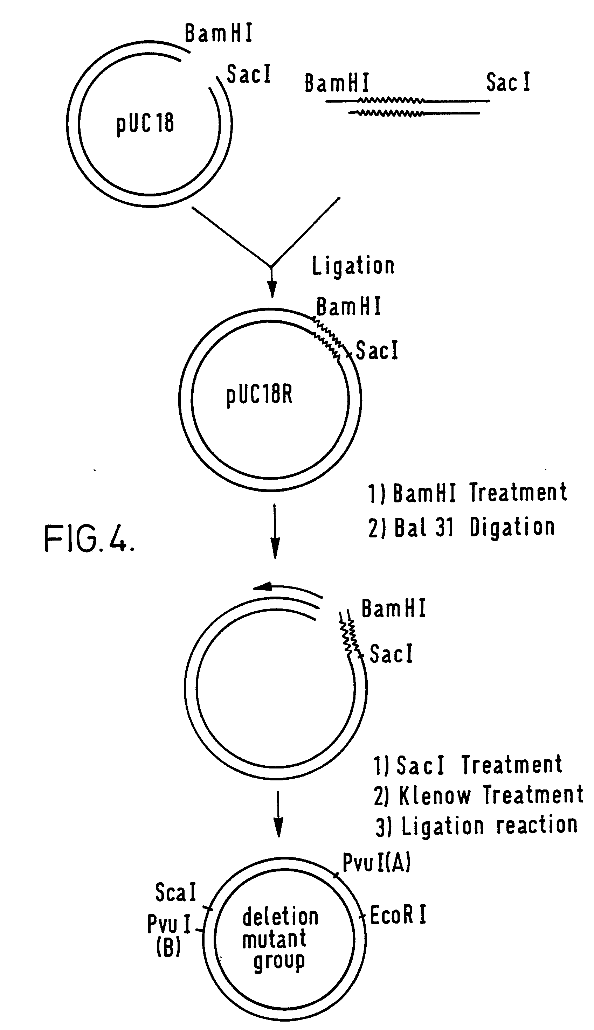

Next, in order to apply the results of the above experiments to the actual production of deletion mutants in plasmids, an experiment for the deletion was carried out using commercially available plasmid pUC18 (shown in Fig. 3) in accordance with the procedure shown in Fig. 4.

Double digestions of pUC18 were carried out with both enzymes at the BmaH I and Sac I sites in multicloning sites of pUC18. Double stranded oligomer (prepared from compounds V and VI) of 5′-phosphorylated chimeric oligomers having the respective cleavage ends was ligated by a ligation reaction into the sites of linearized pUC18. In order to remove any excess of chimeric oligomers V and VI, the reaction solution was treated with Sepharose CL-4B (Pharmacia), whereupon the plasmid fractions were pooled and subjected to the next step as pUC18R (see Example 7 below).

Next, cleavage was carried out with the restriction enzyme BamH I. Using 0.5 units of Bal 31 nuclease based on about 4 pmoles of linearized pUC18R, digestion was carried out at 30°C and sampling was carried out at 0, 10, 30, 45 and 60 minutes (see Example 8 below).

In order to analyze the digestion reaction using Bal 31 nuclease, each sample was treated with Sac I and further treated with Pvu I (which had two sites of A and B), followed by electrophoresis on 1.5% agarose gel. By ethidium bromide staining, the degree of digestion of each band was examined (see Example 9 below and Figure 8).

In the case of the sample not treated with Bal 31 (Figure 8, lane 5), five bands were observed and identified to be 2675 (BamH I - Sac I fragment), 2522 (Pvu I (A) - Sac I fragment), 1620 (Pvu I(B) - Sac I fragment), 1059 (Pvu I (B) - BamH I fragment) and 896 (Pvu I (A) - (Pvu I (B) fragment) from their sizes (thus indicating that the Pvu I treatment was not complete).

In the case of the sample treated with Bal 31 for 10 minutes (lane 6), bands 2675 and 1059 with BamH I site disappear but bands 2522, 1620 and 896 with Sac I site do not disappear.

With the passage of time, the digestion proceeds up to Pvu I (A) site. In the case of the sample when 60 minutes had passed, the 1620 band with Pvu I (V) site - Sac I site strongly remained, notwithstanding the fact that the other bands disappear (cf. Figure 8, lanes 6 to 9). These results show that the digestion with Bal 31 nuclease proceeds from the BamH I site but the Sac I site remains undigested.

Next, after the ends of the Sac I-treated samples obtained in the experiment described above were made blunt by Klenow fragments in the presence of four deoxynucleotide triphosphates (dNTPs), the samples were cyclized by a ligation reaction and E. coli JM103 (a publically-available microorganism) was transformed thereon in a conventional manner. Two clones from the 0 minute sample and five clones from the 10, 30, 45 and 60 minutes samples were selected at random. Next, the clones were treated with a restriction enzyme in multicloning site of pUC18R and the presence or absence of the site was examined by the presence or absence of linearized plasmid by 1.2% agarose gel electrophoresis (see Example 10, Figure 9 and Table 2). As shown in Table 2 and Figure 9, the clones were classified into Type C in which the EcoR I site and the Xba I to Hind III sites all remained, Type D in which both the EcoR I site and the Xba I to Hind III sites had disappeared, Type A in which the EcoR site remained and the Xba I to Hind III sites disappeared and Type B in which the Xba I site alone disappeared.

It is suggested that Type C was obtained either by incomplete treatment so that Bal 31 did not proceed in both directions (Table 2, clone 60-1) or by not being treated with Bal 31 (Table 2, clones 0-1, 0-2) and Type D was obtained because the ligation of the chimeric oligomers was incomplete (Table 2, clones 45-1, 45-4, 45-5 and 60-2. Other Type A (B) indicates that the EcoR I site is stored and deletion occurs at the Xba I site and indicates that the digestion with Bal 31 proceeds in the desired direction. Furthermore, and in contrast, no clone wherein the EcoR I site has disappeared can be said that the direction of deletion can be controlled as desired.

Next, in order to examine the length of deletion of DNA, 2 clones which were obtained by Bal 31 treatment for 45 and 60 minutes were subjected to EcoR I and Sca I double digestions and the size of the cleaved fragments was examined on agarose gel. Bands of about 520 and 460 bp were observed respectively. This indicates that by subtracting from the band of 944 bp to be obtained from the clone untreated with Bal 31, deletion of at least 400 to 500 bp (because of screening as Amp$^r$) occurs by Bal 31 treatment for 45 to 60 minutes (Figure 10, lanes 4 to 6).

From the foregoing experimental results, it is that a method for the deletion with Bal 31 nuclease in only one direction in the case of DNA fragments or on a plasmid level using the chimeric oligomer has been established and the chimeric oligomer can be used for this purpose.

The chimeric oligomer used herein contains seven base pairs of O-methyl RNA but at least one base may be sufficient. Considering the production costs, even chimeric oligomers having a relatively long length containing a GC base pair can sufficiently achieve the purpose. Furthermore, the site of restriction enzyme used in the DNA part may be initiated immediately from the 2$'$-O-methyl RNA part. If chimeric oligomers containing a plurality of restriction enzyme sites in the X and Y regions described above are prepared, cleavage can be effected by some restriction enzymes depending upon the intended purpose and, by applying the concept of linker or adaptor in DNA engineering, the chimeric oligomer can be used for general purposes.

As described above, the principle of the present invention has been proven experimentally. Therefore, it can be said that the present invention provides a new method which is extremely effective and simple in the deletion of cloned genes.

EXAMPLE 1 Preparation of chimeric oligonucleotide (Preparation of 5$'$-GATCCGmGmAmUmCmCmCmGGGGTCGACGAGCT-3$'$ (V) wherein m represents an O-methylnucleotide unit and the other letters represent DNA units)

One micromole of 5$'$-dimethoxytritylthymidine- linked Controlled Pore Glass Cartridge Column (manufactured by Applied Biosystems Co., Ltd.) was attached to a DNA Synthesizer Model 380A (manufactured by Applied Biosystems Co., Ltd.). According to the operations shown in Table 3A, a protected chimeric oligomer was synthesized using phosphoramidite in the order Ic, IIIc, IIc, IIIc, Ic, IIc, IIIc, Ic, IVc, IIIc, IIIc, IIIc, IIIc, Ib, Ib, Ib IVb, IIb, IIIb, Ic, Ic, IVc, IIc and IIIc, using 25 cycles. Next, by the operations shown in Table 3B, the chimeric oligomer was excised from the solid phase support (CPG) as an ammonium hydroxide solution in a 3 ml vial and heated at 55°C for 9 hours in an autoclave to remove the beta-cyanoethyl group of the phosphate and acyl groups of base moiety. After cooling, the ammonia was removed. The residue obtained was dissolved in 400 microlitres of 0.1 M acetate-triethylamine buffer (TEAA) (pH 7.0) containing 10% acetonitrile and half was applied to a 13 mm ⌀ x 150 mm column packed with reversed phase silica gel (Waters, Prep Pak-500/C-18). Column chromatography was performed using 0.1 M TEAA (pH 7.0) with linear gradient elution using 10 to 35% acetonitrile as the mobile phase. A quantitative determination was performed by absorbance at 254 nm to isolate 17 OD units of 5$'$-dimethoxytrityl chimeric oligonucleotide. After the solvent had been evaporated in vacuo, 3 ml of 80% aqueous acetic acid solution was added and the mixture was allowed to stand at room temperature for 10 minutes. After the solvent had been evaporated in vacuo, the residue was co-evaporated with water to remove acetic acid. The residue was dissolved in water. After being washed with ethyl acetate, the solution was evaporated in vacuo. The product was purified by high performance liquid chromatography. The chromatography was performed at a flow rate of 1.2 ml/minute using 0.1 M TEAA (pH 7.0), by linear gradient elution using 5 to 25% acetonitrile as the mobile phase on a YMC Pack ODS column (manufactured by Yamamura Chemical Co., Ltd.). The eluted main peak was pooled to give chimeric oligonucleotide in an amount of 5.2 OD units (above 20 nmols). By similar operations, the oligomer 5$'$-CGTCGACCCCGmGmGmAmUmCmCmG-3$'$ (compound VI) was obtained in an amount of 3.1 OD (17 nmols).

## EXAMPLE 2

### 5'-$^{32}$P phosphate labeling

250 microcurie of gamma-$^{32}$P-ATP (Amersham Co., Ltd., > 5000 curie/mmol) was added to 5 microlitres (10 nmols) of 2 mM ATP. After the mixture had been evaporated in vacuo to dryness, 160 microlitres of water were added to form an ATP solution for labeling (250 microcurie/10 nmole/160 microlitres). To an Eppendorf tube, 1 nmole of Compound VI was added. After the solution had been evaporated in vacuo to dryness, 32 microlitres of the ATP solution, 5 microlitres of 0.5 M Tris HCl (pH 7.5), 5 microlitres of 0.1 M MgCl$_2$ and 5 microlitres of 0.1 M DTT were added to form a solution. To the solution was added 1.5 microlitres (10 U, TAKARA) of T4-polynucleotide kinase and reaction was allowed to take place at 37°C for 40 minutes. Next, 1.5 microlitres (10 U) of kinase was added and the reaction was continued for further 30 minutes. By heating at 95°C for 5 minutes, the reaction was stopped.

In the case of Compounds VI, VII and VIII, reactions were conducted in a similar manner to obtain 5'$^{32}$P labeled products.

## EXAMPLE 3

### Preparation of Dimers from Monomers (1) to (8) by ligation

To the 5'-$^{32}$P labeled oligomer V (400 pmoles/20 microlitres of the reaction solution described above, as obtained in Example 2), there were added 400 pmoles/20 microlitres of unlabeled oligomer (Compound VI), 50mM Tris HCl (pH 7.5) and 10 mM MgCl$_2$. The mixture was gradually cooled from 80°C to 25°C over about 3 hours. Subsequently, the mixture was put on a water bath at 16°C and then 5 microlitres of 0.1 M DTT, 5 microlitres of 2 mM ATP and 1 microlitre (175 U, TAKARA) of DNA ligase were added to the mixture (51 microlitres in total) and the mixture was allowed to stand for 2 hours.

The same amount of water was added to the reaction solution. After being extracted with phenol and then with chloroform followed by ethanol precipitation, the residue was evaporated in vacuo to dryness. The whole amount was dissolved in 50 microlitres of water, 10 microlitres of which was transferred to an Eppendorf tube and subjected to Cerenkov counting (Example 4). Then, 0.5 microlitres of the solution was pooled and subjected to 20% polyacrylamide gel electrophoresis. By autoradiographic analysis, the formation of a dimer from the monomer (1) was confirmed. With respect to the other dimers (those formed from (2) to (8)), they were obtained in the same manner and subjected to analysis (see Figure 5).

## EXAMPLE 4

From the gamma-$^{32}$P-ATP solution (2000 pmoles/32 microlitres) used in Example 2, 1 microlitre (62 pmoles) was taken and put in an Eppendorf tube and subjected to Cerenkov counting using a liquid scintillation counter. Furthermore, 61 microlitres of water were added to dilute the solution and 1 microlitre (1 pmole) of the solution was taken and subjected to Cerenkov counting. The values were 121 x 10$^4$ and 2.1 x 10$^4$ cpm respectively. Therefore, the specific radioactivity of gamma -$^{32}$P-ATP is 2.0 x 10$^4$ cpm/pmole ATP. Therefore the results measured with 10 microlitres of the dimer solutions pooled as in Example 3, are as follows.

$$(1) \quad 162 \times 10^4 \text{ cpm/10 microlitres } (80 \text{ pmoles})$$
$$(2) \quad 114 \times 10^4 \quad " \quad (57 \text{ pmoles})$$
$$(3) \quad 119 \times 10^4 \quad ". \quad (59 \text{ pmoles})$$
$$(4) \quad 103 \times 10^4 \quad " \quad (51 \text{ pmoles})$$
$$(5) \quad 152 \times 10^4 \quad " \quad (76 \text{ pmoles})$$
$$(6) \quad 11.7 \times 10^4 \quad " \quad (58 \text{ pmoles})$$
$$(7) \quad 136 \times 10^4 \quad " \quad (68 \text{ pmoles})$$
$$(8) \quad 107 \times 10^4 \quad " \quad (53 \text{ pmoles})$$

Accordingly, the solution contains about 50 to 80 pmoles (condensed to monomer) of oligomer.

Again 10 microlitres of each of the dimer solutions was pooled and 40 microlitres of water were added thereto, for dilution. Each diluted solution was used as a raw reaction solution in the following step.

EXAMPLE 5

Cleavage of dimer and monomer double strands with restriction enzyme

4 microlitres of each of the double stranded dimer solutions (including monomers) prepared in Example 4 were out into Eppendorf tubes and (5 x) 2 microlitres of buffer for restriction enzyme was added thereto. To each was added 4 microlitres of restriction enzyme (48 U of BamH I, 20 U of Sma I, 24 U of Hinc II, 40 U of Sac I, TAKARA). The reaction with BamH I and Sma I was conducted at 30°C and the reaction with Hinc II and Sac I was conducted at 37°C, each for 40 minutes.

5 microlitres of the reaction solution was taken and a gel-loading buffer containing a dye mixture was added thereto. The mixture was subjected to 20% polyacrylamide electrophoresis and analyzed by autoradiography (see Figure 6).

## Composition of 5 x Buffer

| BamH I | Sma I |
|---|---|
| 50mM Tris HCl (pH8) | 50mM Tris HCl (pH8) |
| 35mM $MgCl_2$ | 35mM $MgCl_2$ |
| 500mM NaCl | 100mM KCl |
| 35mM DTT | 35mM DTT |
| Hinc II | Sac I |
| 50mM Tris HCl (pH8) | 50mM Tris HCl (pH8) |
| 35mM $MgCl_2$ | 35mM $MgCl_2$ |
| 300mM NaCl | 35mM DTT |
| 35mM DTT | |

EXAMPLE 6

Digestion with Bal 31 nuclease

20 microlitres of each of the double stranded dimer solutions (also including monomers) prepared as in Example 4 were put into an Eppendorf tube and 10 microlitres of 5 x buffer for Bal 31 nuclease digestion and 15 microlitres of water were added thereto for dilution. From each solutions, 4.5 microlitres each was taken and 5 micrlitres of 50 mM EDTA solution were added to form a 0 minute sample. Next, 4.5 microlitres (0.234 U, obtained by diluting 2.6 U/microlitres of TAKARA Bal 31 enzyme solution 50 times) of Bal 31 enzyme solution was added to the solution and reaction was allowed to take place at 30°C. 5 microlitres of each of the reaction mixtures were taken and reaction was allowed to take place with the passage of time and each was then added to 5 microlitres of ice cooled gel-loading buffer containing 50 mM EDTA to stop the reaction. 5 microlitres of each sample was subjected to 20% polyacrylamide electrophoresis and analyzed by autoradiography (see Figure 7).

## Composition of 5 x buffer:

$$100 \text{ mM Tris HCl (pH 8.0)}$$
$$60 \text{ mM CaCl}_2$$
$$60 \text{ mM MgCl}_2$$
$$5 \text{ mM EDTA}$$
$$3 \text{ M NaCl}$$

## EXAMPLE 7

### Preparation of plasmid (pUC18R) having chimeric oligonucleotide inserted therein

To 14 microlitres (2.8 nmoles) of each of the chimeric oligomers V and VI prepared in Example 1 there were added 4 microlitres of 0.5 M Tris HCl (pH 7.5), 4 microlitres of 0.1 M MgCl₂, 2.8 microlitres of 2 mM ATP, 4 microlitres of 0.1 M DTT, 9.2 microlitres of H₂O and 1 microlitre (7 units) of kinase. After standing at 37°C for 30 minutes, 1 microlitre (7 units) of kinase was also added and the mixture was allowed to stand at 37°C for 30 minutes to complete kination (40 microlitres each in the whole amount). After treatment at 90°C for 3 minutes to inactivate the kinase, the reaction mixtures were combined and the temperature was gradually lowered from 90°C to effect annealing (80 microlitres in total).

Separately, 20 microlitres of Sac I x 5 buffer, 27 microlitres of H₂O and 8 microlitres (86 units) of Sac I were added to 48 microlitres (TAKARA, 50 micrograms: 28.2 pmoles: 1/100 times oligomer) of pUC18. The mixture was allowed to stand for 3.5 hours to effect cleaving. There were then added 2 microlitres of 5 M NaCl and 4 microlitres (48 units) of BamH I. The mixture was allowed to stand at 30°C for an hour and 2 microlitres (24 units) of BamH I was then added thereto and the reaction was completed at 30°C for an hour. After treatment with phenol and chloroform followed by ethanol precipitation and washing with 80% ethanol, the reaction mixture was evaporated in vacuo to dryness. 7 microlitres of 0.5 M Tris HCl (pH 7.5), 7 microlitres of 0.1 M MgCl₂ and 25 microlitres of H₂O were added thereto to dissolve the dry residue. To the solution, there were added 80 microlitres of the phosphorylated double stranded oligomer described above, 15 microlitres of 2 mM ATP, 15 nicrolitres of 0.1 M DTT and 1 microlitre (175 units) of ligase, and the mixture was allowed to stand at 4°C for 16 hours (150 microlitres in total).

After treatment with phenol and chloroform followed by ethanol precipitation and washing with 80% ethanol, the reaction mixture was evaporated in vacuo to dryness. The residue was dissolved in 400 microlitres of H₂O and applied to a column (⌀ : 1 cm, length : 10 cm, buffer : 0.5 M NaCl/TE) containing Sepharose CL-4B (Pharmacia) and the first peak eluted was pooled. After ethanol precipitation, washing with 80% ethanol and evaporation in vacuo to dryness, the residue was dissolved in 500 microlitres of water and the OD was determined to be 0.83 OD (about 41.5 micrograms).

## EXAMPLE 8

### Digestion with Bal 31 nuclease after cleavage of pUC18R with BamH I

After dissolving pUC18R evaporated to dryness in 74 microlitres of $H_2O$, 20 microlitres of BamH I x 5 buffer and 4 microlitres (48 units) of BamH I were added to the residue. After the mixture had been allowed to stand at 30° C for an hour, 2 microlitres (24 units) of BamH I was further added to the mixture and the reaction was completed at 30° C for an hour. After treatment with phenol and chloroform, followed by ethanol precipitation and washing with 80% ethanol, the mixture was evaporated in vacuo to dryness. 500 microlitres of water was added to the residue and the OD was determined to be 0.88 OD (about 44 micrograms). After evaporating in vacuo to dryness, the sample was dissolved in 80 microlitres of 10 mM Tris HCl (pH 8.0) containing 1 mM EDTA. To 13 microlitres of the solution were added 7 microlitres of $H_2O$ and 5 microlitres of Bal 31 x 5 buffer to make the total amount 25 microlitres. The solution was used for digestion with Bal 31 nuclease. From 25 microlitres, 5 microlitres was pooled as a 0 minute sample and added to 5 microlitres of ice-cooled 50 mM EDTA. To the remaining 20 microlitres of solution, there were added 0.5 units/microlitres (obtained by diluting 2.6 units/microlitres of commercially available enzyme 5 times) of Bal 31 and the mixture was allowed to stand at 30° C. After the passage of times of 10, 30, 45 and 60 minutes, samples each of 5 microlitres were added to 5 microlitres of ice-cooled 50 mM EDTA to stop the reaction. To each of sample, there were added 50 microlitres of $H_2O$ and the mixture was subjected to treatment with phenol and chloroform, to ethanol precipitation, washing with 80% ethanol and to evaporation in vacuo to dryness.

## EXAMPLE 9

First analysis of the digestion with Bal 31

Five samples were each dissolved in 16 microlitres of $H_2O$ and 4 microlitres of Sac I x 5 buffer and 4 units of Sac I were added to the solution, followed by reaction at 37° C for an hour. A further 4 units of Sac I were added thereto. After being allowed to stand at 37° C for an hour, the mixture was subjected to treatment with phenol and chloroform, to ethanol precipitation, and to washing with 80% ethanol. The residue was evaporated in vacuo to dryness. The residue was dissolved in 10 microlitres of $H_2O$ 9 microlitres of which were pooled. There were then added 2.5 microlitres of Pvu I x 5 buffer and 2.7 units of Pvu I ,followed by reaction at 37° C for an hour. The reaction mixture was subjected to 1.5% agarose gel electrophoresis and subjected to analysis (see Figure 8).

## EXAMPLE 10

Second analysis of the digestion with Bal 31

The remaining 1 microlitre of the sample cleaved with Sac I was prepared as in Example 9 so as to have a final concentration of 10 mM Tris HCl (pH 7.5), 10 mM $MgCl_2$, 60 mM NaCl, 6 mM DTT and 20 micromolar dNTPs Klenow fragment (TAKARA, 0.8 units). The total amount was 20 microlitres. The mixture was allowed to react at 37° C for an hour. By treatment with phenol and chloroform, by ethanol precipitation, by washing with 80% ethanol and by evaporation in vacuo to dryness, there was obtained a residue to which were added 1 microlitre of 0.5 M Tris HCl (pH 7.5), 1 microlitre of 0.1 M $MgCl_2$, 1 microlitre of 0.1 M DTT, 1 microlitre of 2 mM ATP, 6 microlitres of $H_2O$ and 35 units of and the mixture was allowed to stand at 4° C for 21 hours.

Samples each of 5 microlitres each of the ligation mixture were added to 120 microlitres of competent cells (E. Coli JM103) and samples of 5 microlitres and 120 microlitres were inoculated on a YT plate (Amp, 50 mg/litre) and cultured at 37° C overnight. The number of colonies is as follows.

|  | 5 microlitre sample | 120 microlitre sample |
|---|---|---|
| 0 Minute | 7 | 1008 |
| 10 Minutes | 1 | 372 |
| 30 Minutes | 3 | 152 |
| 45 Minutes | 1 | 100 |
| 60 Minutes | 0 | 14 |

Two samples of the 0 minute sample and 5 samples of each of the 10, 30, 45 and 60 minute samples were selected at random from the plate and precultured on 6 ml of 2 YT (Amp, 100 mg/litre) at 37°C for 7 hours. The culture solution was inoculated as 50 microlitre samples on 5 ml of 2YT (Amp, 100 mg/litre) and by cultured at 37°C overnight. After collecting the cells in a conventional manner, the plasmid was prepared by the alkali-SDS method. Each clone was dissolved in 100microlitre of 10 mM Tris HCl (pH 8.0) containing 1 mM EDTA and 4 microlitres each of which was pooled. One unit of each enzyme, 2 microlitres of each 5 x Buffer and water were added to make the whole volume 10 microlites. After treating with EcoR I, Xba I, Hinc II, Pst I, Sph I, Hind III and Sac I, the reaction mixture was subjected to 1.2% agarose gel electrophoresis and subjected to analysis (see Figure 9 and Table 2).

EXAMPLE 11

Third analysis of the digestion with Bal 31 nuclease

From the clones obtained in Example 10, one clone from 0 minute sample was used as a control. After being treated with EcoR I ani Sac I, one clone from the 45 minute sample and one clone from the 60 minute sample (using 40 microlitres of the 100 microlitres of plasmid solution obtained in Example 10) were subjected to 2% agarose gel electrophoresis and ethidium bromide staining, whereafter the length of digested fragments was analyzed (see Figure 10.)

As has been described above, according to the present invention, selective deletion can occur on a DNA gene by a relatively simple method and deletion mutants in opposite directions can also be produced separately so that the present invention greatly contributes to the field of genetic engineering.

TABLE 1

Structural formula 1

I   X=H      Y=H  Z=OCH$_3$

Ia  X=DMTr  Y=H  Z=OCH$_3$

Ib  X=DMTr  Y=R$_1$  Z=OCH$_3$

Ic  X=DMTr  Y=R$_1$  Z=H

II   X=H      Y=H    Z=OCH$_3$

IIa  X=DMTr  Y=H    Z=OCH$_3$

IIb  X=DMTr  Y=R$_1$  Z=OCH$_3$

IIc  X=DMTr  Y=R$_1$  Z=H

III   X=H      Y=H    Z=OCH$_3$

IIIa  X=MMTr  Y=H    Z=OCH$_3$

IIIb  X=MMTr  Y=R$_1$  Z=OCH$_3$

IIIc  X=DMTr  Y=R$_1$  Z=H

IIId  X-DMTr  Y=P    Z=H

IV   X=H      Y=H    Z=OCH$_3$ R$^1$=H

IVa  X=DMTr  Y=H    Z=OCH$_3$ R$^1$=H

IVb  X=DMTr  Y=R$_1$  Z=H      R$^1$=H

IVc  X=DMTr  Y=R$_1$  Z=H      R$^1$=CH$_3$

IVd  X=DMTr  Y=P    Z=H      R$^1$=CH$_3$

R$_1$ = (iPr)$_2$-N-P(CH$_2$-CH$_2$-CN)

P  - Long chain alkyl linked via succinyl group, CPG

16

Table 2

| Clone | EcoRI | XbaI | HincII | PstI | SphI | HindIII | ScaI | Type |
|-------|-------|------|--------|------|------|---------|------|------|
| 0-1 | + | + | + | + | + | + | + | C |
| -2 | + | + | + | + | + | + | + | C |
| 10-1 | + | - | - | - | - | - | + | A |
| -2 | + | - | - | - | - | - | + | A |
| -3 | + | - | - | - | - | - | + | A |
| -3 | + | - | - | - | - | - | + | A |
| -4 | + | - | - | - | - | - | + | A |
| -5 | + | - | - | - | - | - | + | A |
| 30-1 | + | - | - | - | - | - | + | A |
| -2 | + | - | - | - | - | - | + | A |
| -3 | + | - | - | - | - | - | + | A |
| -4 | + | - | - | - | - | - | + | A |
| -5 | + | - | - | - | - | - | + | A |
| 45-1 | - | - | - | - | - | - | nd | D |
| -2 | + | - | - | - | - | - | + | A |
| -3 | + | - | - | - | - | - | + | A |
| -4 | - | - | - | - | - | - | nd | D |
| -5 | - | - | - | - | - | - | nd | D |
| 60-1 | + | + | + | + | + | + | nd | C |
| -2 | - | - | - | - | - | - | nd | D |
| -3 | + | - | - | - | - | - | + | A |
| -4 | + | - | + | + | + | + | nd | B |
| -5 | + | - | - | - | - | - | + | A |

+ ... site cut          nd ... not done

- ... site not cut

EP 0 320 103 A2

Table 3A        1 Cycle of Operation for Strand
                Extension Reaction (using 1 micromolar
                CPG resin)

| Reagent | Time for Feeding Liquid (sec), | Frequency |
|---|---|---|
| Washing with $CH_3CN$ | 20 | |
| Air Drying | 5 | |
| 3% $CCl_3COOH/CH_2Cl_2$ | 50 | |
| Washing with $CH_3CN$ | 60 | |
| Air drying | 5 | |
| Washing with $CH_3CN$ | 60 | |
| Air drying | 20 | |
| Phosphoramidite/tetrazole/$CH_3CN$ | 12 | |
| Allowing to stand | 30 | |
| Air drying | 5 | |
| $Ac_2O$-DMAP-luthidine/THF | 20 | |
| Allowing to stand | 15 | |
| Air drying | 10 | |
| Washing with $CH_3CN$ | 15 | |
| Air drying | 5 | |
| $I_2$-$H_2O$-luthidine/THF | 23 | |
| Allowing to stand | 30 | |
| Air drying | 20 | |
| Washing with $CH_3CN$ | 20 | x 4 |
| Air drying | 5 | x 4 |

Table 3B        Operation for Excision from CPG Resin
                (using 1 micromolar resin)

| Reagent | Time for Feeding Liquid (sec), | Frequency |
|---|---|---|
| Air drying | 60 | |
| Conc. $NH_3$ | | |
| (subsequent operations are | | |
| performed by feeding to a | 15 | x 4 |
| sample vial via CPG resin) | | |
| Allowing to stand | 900 | x 4 |
| Air drying | 15 | |
| Conc. $NH_3$ | 15 | |
| Air drying | 15 | |

## Claims

1. A single-stranded linear polynucleotide for use in the deletion of DNA from a gene, having the general formula I or I':

$5'$-A-$(B)_m$-X-$(C)_n$-D-$3'$      (I)

or

$5'$-A-$(B)_m$-X-$(C)_n$-$3'$      (I')

wherein A and D are each, independently, (i) a terminal DNA sequence as formed in one of the single strands of a double-stranded sequence of base-pairs at the cleavage site of a restriction enzyme recognition sequence or (ii) a DNA linker sequence from which a terminal DNA sequence as described in (i) above may be generated;

wherein B and C are each, independently, chosen from (a) one of the single-stranded parts of a double-stranded sequence of deoxyribonucleotide base pairs which constitutes a restriction enzyme recognition sequence, optionally coupled at either end with an integral number of additional deoxyribonucleotides chosen from A, C, G and T, and (b) any sequence of deoxyribonucleotides; with the proviso that, in the polynucleotide of general formula I or I' there is present at least one sequence representing B or C which is in accordance with the definition given in (a) above;

and X includes a sequence of nucleotides which, in double-stranded form, is resistant to digestion by a DNA nuclease;

and wherein m and n are each, independently, zero or an integer of from 1 to 5.

2. A single-stranded linear polynucleotide for use in the deletion of DNA from a gene, having the general formula I or I';

$5'$-A-$(B)_m$-X-$(C)_n$-D-$3'$      (I)

or

$5'$-A-$(B)_m$-X-$(C)_n$-$3'$      (I')

wherein A and D are independently chosen from

(i) a single-stranded linker of deoxyribonucleotides having the general formula II or II'

$R_5$-W-$R_6$      (II) or W      (II')

in which each of $R_5$ and $R_6$ represents a sequence of the same length comprising 1, 2 or 3 nucleotides chosen from G (guanine) and C (cytosine) and W is a sequence comprising from 4 to 6 nucleotides chosen from G, C, A (adenine) and T (thymidine) or W is a sequence comprising 7 nucleotides chosen from G, C, A and T, the terminal nucleotides of which sequence are chosen from G and C; with the proviso that the sequence of nucleotides in the linker of general formula II or II' is chosen such that, when it is base-paired with its complementary DNA sequence, there is formed a palindromic sequence of base pairs; and

(ii) a terminal DNA sequence of from one to six nucleotides formed by cleavage between two adjacent nucleotides of sequence W as defined above;

wherein B and C are independently chosen from

(a) a single-stranded length of deoxyribonucleotides of sequence W as defined in relation to general formula II above, optionally coupled at either end with an integral number of additional deoxyribonucleotides chosen from A, C, G and T; and

(b) any sequence of deoxyribonucleotides; with the proviso that in the polynucleotide of general formula I or I' there is present at least one sequence representing B or C which is in accordance with the definition given in (a) above;

and X represents a sequence of nucleotides which, in double-stranded form, is resistant to digestion by a DNA nuclease;

and m and n are each, independently, zero or an integer of from 1 to 5.

3. A single-stranded polynucleotide for use in the deletion of DNA from a gene, having the general formula I or I':

$5'$-A-$(B)_m$-X-$(C)_n$-D-$3'$      (I)

or

$5'$-A-$(B)_m$-X-$(C)_n$-$3'$      (I')

wherein A and D are each, independently, represented by a linker sequence (of general formula II or II' below) or a terminal DNA sequence (of general formula III below) as formed in one of the single strands of a double-stranded sequence of base pairs at the cleavage site of a restriction enzyme recognition sequence;

19

B and C are each, independently, represented by (a) a single-stranded part (of general formula II below) of a double-stranded sequence of deoxyribonucleotide base pairs which constitutes a restriction enzyme recognition sequence, optionally coupled at either end with an integral number of additional deoxyribonucleotides chosen from A, C, G and T or (b) any sequence of deoxyribonucleotides; with the proviso that, in the compound of formula I or I', there is present at least one sequence representing B or C which is in accordance with the definition given in (a) above; wherein X represents a sequence of nucleotides which, in double-stranded form, is resistant to digestion by a DNA nuclease; and m and n are each, independently, zero, or an integer of from 1 to 5;

said linker sequence in general formula I being the same as the single-stranded part of a restriction enzyme recognition sequence (general formula II) or represented by general formula II':

W      (II)

$R_5$-W-$R_6$     (II')

wherein $R_5$ and $R_6$ each represents a sequence of the same length comprising 1, 2 or 3 deoxyribonucleotides composed of G (guanine) or C (cytosine); and $R_5$ and $R_6$ are chosen to be complementary when the sequence represented by general formula II' forms double strands;

said single-stranded part of a restriction enzyme recognition sequence of general formulae II being selected from the following 8 sequence groups:

$$R_1R_2R_3R_4, \qquad R_1R_2NR_3R_4,$$
$$AR_1R_1R_3R_4T, \qquad CR_1R_2R_3R_4G,$$
$$GR_1R_2R_3R_4C, \qquad TR_1R_2R_3R_4A,$$
$$CR_1R_2NR_3R_4G \text{ and } GR_1R_2NR_3R_4C$$

wherein the sequential order of $R_1$, $R_2$, $R_3$ and $R_4$ comprises, irrespective of the presence or absence of N, the following 16 sequence groups, reading from the left:

AATT, ACGT, AGCT, ATAT,

CATG, CCGG, CGCG, CTAG,

GATC, GCGC, GGCC, GTAC,

TATA, TCGA, TGCA, TTAA,

and N represents a deoxynucleotide unit of G, C, A (adenine) and T (thymidine),

said single-stranded part of the terminal DNA sequence generated at the cleavage site of a restriction enzyme recognition site being composed of a sequence group represented by general formula III:

$R_1R_2R_3R_4$,

$R_2R_3R_4$, $R_3R_4$, $R_4$,

$R_2NR_3R_4$, $NR_3R_4$, $R_3R_4$, $R_4$

$R_1R_2R_3R_4Y$, $R_2R_3R_4Y$, $R_3R_4Y$, $R_4Y$, Y,

$R_1R_2NR_3R_4Z$, $R_2NR_3R_4Z$, $NR_3R_4Z$, $R_3R_4Z$, $R_4Z$, Z,

$R_1R_2R_3$, $R_1R_2$, $R_1$,

$R_1R_2NR_3$, $R_1R_2N$, $R_1R_2$, $R_1$,

$YR_1R_2R_3R_4$, $YR_1R_2R_3$, $YR_1R_2$, $YR_1$, Y

$ZR_1R_2NR_3R_4$, $ZR_1R_2NR_3$, $ZR_1R_2N$, $ZR_1R_2$, $ZR_1$, Z

in which Y represents a deoxyribonucleotide unit of G, C, A and T; Z represents a deoxyribonucleotide unit of G and C, and N has the same definition as described above.

4. A single-stranded polynucleotide according to any one of the preceding claims, wherein the region X includes a sequence of up to 50 nucleotides which, in a double-stranded form, is resistant to Bal 31 nuclease.

5. A single-stranded polynucleotide according to claim 4, wherein the region X includes one or more ribonucleotide or derivative thereof which is resistant to Bal 31 nuclease, for example a 2'-O-methyl ribonucleotide.

6. A compound for DNA deletion, comprising two or more polynucleotides represented by the general formula I or I' as defined in any one of claims 1 to 5 linked in tandem.

7. A double-stranded oligomer comprising a primary strand of general formula I or I' as defined in any one of claims 1 to 5 base-paired with its complementary DNA strand in which the region of the complementary strand which is base-paired with the X-region of the primary strand optionally comprises a sequence which is resistant to digestion by a DNA nuclease;

wherein, if the sequence of nucleotides represented by A and D in the primary strand of formula I or I' is a terminal DNA sequence, the corresponding terminal sequence of the complementary strand of the oligomer is chosen such that the respective end of the oligomer corresponds to the end formed at the cleavage site of a restriction enzyme recognition sequence.

8. A double-stranded oligomer for DNA deletion, comprising two or more oligomers as claimed in Claim 7 directly linked in tandem.

9. A method for producing a compound for DNA deletion comprising generating, from a double-stranded oligomer in accordance with claim 7 or 8 which has one or two terminal linker sequence, an oligomer according to claim 7 or 8 in which the respective end or ends of the oligomer correspond, independently, to the end formed at the cleavage site of a restriction enzyme recognition sequence, and subsequently ligating one or both of the ends of the thus formed oligomer to the corresponding end or ends of a desired double-stranded DNA fragment.

10. A method for producing a compound for DNA deletion comprising ligating, to the corresponding end or ends or a desired double-stranded DNA fragment, one or both of the ends of an oligomer in accordance with claim 7 or 8 in which one or both of the ends of the oligomer correspond, independently, to the end formed at the cleavage site of a restriction enzyme recognition sequence.

11. A method of DNA deletion comprising subjecting to deletion by a DNA nuclease a compound as produced by the method of Claim 9 or 10 in which one only of the ends of the oligomer is ligated with the corresponding end of the desired double-stranded DNA fragment; or, if both ends of the oligomer are ligated with the corresponding ends of the desired double-stranded DNA fragment, first cleaving the compound as produced by the method of Claim 9 or 10 with a restriction enzyme at one of the regions A, B, C or D containing a restriction site, and second subjecting the resulting derivative to deletion by a DNA nuclease.

12. A method according to claim 11, wherein the DNA nuclease is Bal 31 nuclease.

FIG.1.

Step 1

X        Y

Ligase

∿∿∿    :0-Methyl RNA

——    : DNA

Step 2

Cleavage at Y region

Bal 31 nuclease treatment

Step 3

Cleavage at X or Z region

Klenow  treatment

Ligation reaction

B-part  deletion mutants

# FIG.2.

Z

A     B

1      2

# FIG.3.

PvuI (B)
2066

ScaI
2177

Amp^r

pUC18
2686 bp

LacZOP

PvuI (A)
276

Hind II 399
Sph I 405
Pst I 411
Sal I, Acc I, Hinc II 417
Xba I 423
BamH I 429
Sma I, Xma I 434
Kpn I 438
Sac I 444
EcoR I 450

FIG.4.

FIG.5.

FIG.6A.

FIG.6B.

FIG.7A.

FIG.7B.

2  25 26 27 28  4  29 30 31 32  6  33 34 35 36  8  37 38  39 40

② ④ ⑥ ⑧

EP 0 320 103 A2

FIG.8.

9  8  7  6  5  4  3  2  1

2322
2027
1353
1078
872
603

FIG.10.

6  5  4  3  2  1

2322
2027
1353
1078
872

587
540
504
458
434

FIG.9.